# EUROPEAN PATENT SPECIFICATION

(11) **EP 2 908 898 B1**
(45) Date of publication and mention of the grant of the patent: **26.07.2023**
(21) Application number: 13717994.1
(22) Date of filing: 13.03.2013
(51) Int. Cl.: A61M 25/00

(54) **VAPOR HYDRATED MEDICAL DEVICE WITH LOW SURFACE ENERGY SLEEVE**
DAMPFHYDRIERTE MEDIZINISCHE VORRICHTUNG MIT HÜLSE MIT NIEDRIGER OBERFLÄCHENENERGIE
DIPOSITIF MÉDICAL HYDRATÉ PAR VAPEUR AYANT UNE MANCHE DE TENSION DE SURFACE RÉDUITE

(30) Priority: 18.10.2012 US 201261715370 P
(43) Date of publication of application: 26.08.2015
(73) Proprietor: Hollister Incorporated, Libertyville, IL 60048 (US)
(72) Inventor: ROSTAMI, Shamsedin, Oxford OX1 5DR (GB)
(74) Representative: FRKelly
(86) International application number: PCT/US2013/030898
(87) International publication number: WO 2014/062223

(56) References cited:
- EP-A1- 2 060 296
- EP-A1- 2 277 554
- WO-A2-2005/014055
- WO-A2-2008/046023
- US-A- 4 613 544
- US-A1- 2008 091 145

## Description

### Related Application

This application claims the benefit of and priority to U.S. Provisional Patent Application Serial No. 61/715,370, filed October 18, 2012.

### Field of the Disclosure

This disclosure relates generally to packaging for medical devices that require hydration or wetting prior to use, such as hydrophilic urinary catheters, and more specifically, to packaging that achieves hydration or wetting of a medical device contained in a sleeve or compartment in such a manner that, upon withdrawal of the device from a vapor and liquid impermeable outer wrapper, an exterior of the sleeve or compartment within which the medical device is disposed is drier to the touch.

### Background

While pre-existing medical devices, such as intermittent catheters, have been hydrated prior to use (i.e., having their hydrophilic coatings activated and maintaining the device in a moist condition), such as by opening a sealed sachet of sterile liquid and pouring the liquid onto the device, providing sufficient liquid water in the package (either in the same compartment as the catheter or, at least initially, in a separate compartment) to immerse the device, or filling a package containing the device with water from a faucet or dipping the device in water prior to use, all of these tend to result in undesirable dripping. Other drawbacks of such pre-existing medical devices, specifically intermittent catheters, include the need for the user to wait on the order of 30 seconds or more from the time the catheter comes into contact with liquid until the catheter is ready for insertion, and many patients with limited dexterity have difficulty manipulating the packaging to open separate liquid-containing sachets or compartments to expose the catheter to liquid and/or avoid spillage of liquid from the package onto their clothing.

US Patent Nos. 7,380,658, 7,770,726, 7,886,907, 8,011,505, 8,051,981, and 8,205,745 disclose packaging for medical products, such as hydrophilic intermittent catheters, wherein at least the insertable length of the catheter is hydrated prior to removal of the catheter from a foil outer wrapper. Rather than exposing the catheter to liquid, the systems of these patents exploit a change of phase of water from liquid to vapor that occurs within a liquid and vapor-impermeable package. The hydrophilic catheter surface is activated (and therefore lubricious to facilitate insertion or withdrawal thereof to or from the urethra of a user) by water vapor resulting from the change of phase of the liquid water that is disposed in the package in a manner isolating it from direct contact with the catheter.

It has been found that where catheters are hydrated in such a manner, and the catheter is provided in a vapor permeable sleeve through which the water vapor can pass to reach the hydrophilic catheter surface, the sleeve may be considered undesirably wet by some users when the catheter is removed from the packaging. This disclosure provides a solution to reduce or eliminate sleeve wetness at the point of use.

### Summary of the Disclosure

In order to reduce the wetness or sliminess exhibited by an exterior of a sleeve or compartment provided about a medical device upon removal of the sleeve or compartment from a foil package that, at least initially, contained a quantity of liquid water isolated from the catheter, a sleeve is constructed of one or more liquid impermeable, vapor permeable membrane(s) that permits water molecules to travel from outside the sleeve or compartment to the outer surface of the medical device, e.g., the hydrophilic surface of the catheter, but does not allow water molecules to accumulate on its surface, hence providing a drier-to-the-touch feel to the sleeve's exterior at the point of use. As used herein, water vapor permeable means having a water vapor permeability (moisture vapor transmission rate) greater than 300 g/m²/day, greater than 500 g/m²/day, greater than 1000 g/m²/day, greater than 2000 g/m²/day or preferably greater than 3000 g/m²/day, as measured according to ASTM E-96 Procedure E - Desiccant Method at 100°F (37.8°C) and 75% Relative Humidity.

As used herein, the term low surface energy sleeve refers to a sleeve that is resistant to accumulation of water molecules on the surface thereof. Such sleeves may have an exterior surface wherein the contact angle with liquid water on the exterior surface of the sleeve is at or above 100°. In one example, the liquid water contact angle of the surface may be between about 100° and about 120°. Additionally, the sleeve may have a surface tension below about 40 mN/m (dyne/cm) at 20°C and, preferably, below about 36 mN/m at 20°C. The surface tension of the sleeve may be in the range of about 20 mN/m to about 30 mN/m at 20°C, or preferably, in the range of about 15 mN/m to 36 mN/m at 20°C. Examples of low surface energy polymers are given in Section VI/411 of Polymer Handbook, Third Edition, J. Brandrup and E.H. Immergut.

The sleeve may have any combination of the above-described permeability, surface energy and surface tension properties. For example, the sleeve may have a water vapor permeability that is greater than 500 g/m²/day or greater than 1000 g/m²/day and the exterior surface of the sleeve that is touched by the user has a contact angle with liquid water that is above about 100°. In another embodiment, the water vapor permeability of the sleeve may be greater than 300 g/m²/day, greater than 500 g/m²/day, greater than 1000 g/m²/day, greater than 2000 g/m²/day or preferably greater than 3000 g/m²/day, as measured according to ASTM E-96 Procedure E - Desiccant Method at 100°F (37.8°C) and 75% Relative Humidity and the surface tension may be below about 40 mN/m at 20°C or below about 36 mN/m at 20°C.

It is found that by fabricating the sleeve or compartment using a membrane selected from the low surface energy family of polymers, such as polytetrafluoroethylene (PTFE) and other fluoropolymers, and/or polyolefins, preferably laminated to a non-woven fabric such as pressed polyethylene or PET fibers, wetness or sliminess of the sleeve or compartment upon removal from a foil package can be substantially reduced. In one embodiment, the membrane, sleeve or compartment is comprised of PTFE and/or polyethylene. The polyethylene may be treated by any suitable process to increase or enhance the vapor permeability of the polyethylene. Such processes may include, for example, hole punching by mechanical means, radiation or accelerated ionic particles. Polyethylene also may be blended with one or more other suitable polymers. The polyethylene and other polymers may be made or stretched such that the interface between the polyethylene matrix and the other polymer produces sufficient porosity for water vapor to be transported therethrough. Alternatively, the polyethylene may be made with mineral fillers in conjunction with or instead of additional polymers. For example a calcium carbonate filled polyethylene films may be stretched to create porosity at the interface between the polyethylene and the calcium carbonate particles for the purpose of enhancing water vapor permeability. Still other methods include stretching films of polyethylene to the extent that holes are created at the interface between its crystalline and amorphous regions.

A method for quantifying the reduction of sleeve or compartment wetness has been developed and is disclosed herein.

### Brief Description of the Several Views of the Drawing

Fig. 1 is a side elevational view of a medical device in the form of a hydrophilic catheter disposed in a sleeve or compartment formed of a low surface energy sleeve membrane, within a foil package;
Fig. 2 is a front plan view of the packaged catheter of Fig. 1;
Fig. 3 is an enlarged view of the broken-lined region designated 3 of Fig. 1;
Fig. 4 is a side elevational view of a medical device in the form of a hydrophilic catheter disposed in a sleeve or compartment formed of a bidirectionally vapor permeable membrane, within a foil package;
Fig. 5 is a front plan view of the packaged catheter of Fig. 4;
Fig. 6 is an enlarged view of the broken-lined region designated 6 of Fig. 4.

### Detailed Description of the Preferred Embodiments

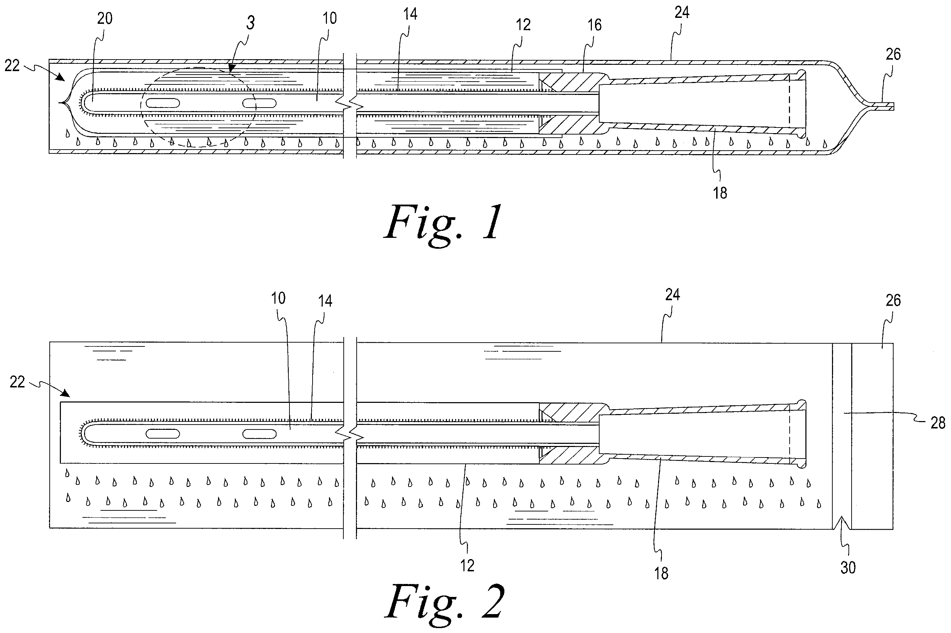

With reference to drawing figures 1-3, a medical device in the form of a catheter 10, such as an intermittent urinary catheter, has at least an insertable portion disposed within a sleeve or compartment 12. Throughout the following discussion, it will be understood that the medical device could comprise any number of different devices including not only catheters but also devices used in reconstructive, cardiovascular, gastrointestinal, otorhinolaryngology, ophthalmological, and urogynecology applications wherein the device is disposed within a sleeve or compartment that is either sealed or unsealed. However, the detailed description will be provided in connection with one particularly advantageous application; namely, a catheter 10 disposed within a sleeve 12 and having a hydrophilic coating 14.

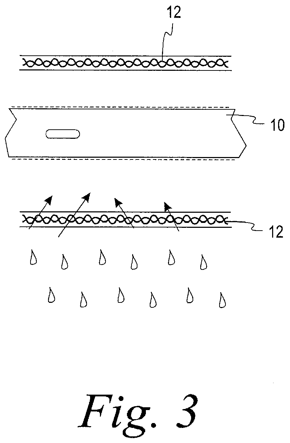

The sleeve or compartment 12 is illustrated as being sealed to a collar 16 of a funnel 18 of the catheter 10. The sleeve 12 may also be sealed to an introducer tip (not shown) at or near a tip end 20 of the catheter 10. However, in figure 1, the sleeve is only sealed to the collar 16, and extends beyond the tip end 20 of the catheter 10. As illustrated in Fig. 3, which is an enlarged view taken along broken-line 3 of Fig. 1, the sleeve 12 is comprised of one or more liquid impermeable, vapor-permeable membranes, wherein each of the membranes permits water molecules to travel from outside the sleeve 12 to the outer hydrophilic surface of the catheter 10, but does not permit accumulation of water molecules on the surface of the sleeve 12.

The membranes forming the sleeve 12 are selected from the low surface energy family of polymers, such as PTFE and other fluoropolymers and/or polyolefins, and could be laminated to a non-woven fabric such as pressed polyethylene or PET fibers. For example, membranes formed of a GORE-TEX^{®} Medical Membrane PTFE and/or polyfluorinated material are found to be suitable for achieving the desired liquid impermeability, preferential vapor permeability, and low surface energy. As used herein, preferential vapor permeability refers to vapor permeability in a first direction, e.g. from an exterior of sleeve 12 toward an interior of sleeve 12, that is greater than a vapor permeability in a second, opposite direction, e.g., from the interior of sleeve 12 toward the exterior of sleeve 12. Preferential vapor permeability may be achieved, as disclosed by W.L. Gore & Associates (for example in its US Patent Nos. 4,194,041) by providing a material, such as PTFE, with a thin, porous fluoropolymer membrane coating with pores that are much larger (on the order of 700 times larger) than a water vapor molecule. This is sometimes referred to as "breathability." Such pores are also much smaller (on the order of 20,000 times smaller) than the size of a water droplet, rendering the membrane impervious to liquid water. Over time, a sufficient amount of water molecules in the form of water vapor traverses the sleeve 12 so as to produce a vapor atmosphere within the interior of the sleeve 12 occupied by the catheter 10 and activate the hydrophilic coating 14. The arrows in figure 3 represent the migration of molecules of water vapor traversing the sleeve 12. As illustrated, the water vapor molecules travel from an exterior of the sleeve 12 to an interior of the sleeve 12, where they can hydrate the coating 14, but due to the low surface energy of the film of which the sleeve 12 is formed, even when the interior of the sleeve 12 reaches 100% humidity, the water vapor molecules do not substantially accumulate at the surface of the sleeve 12 that will be touched by end users.

The sleeve 12 and catheter 10 together constitute a sleeved catheter assembly 22, which is packaged in an outer foil wrapper 24. The foil wrapper 24 is both liquid and vapor impermeable, and may include a heat seal 26 at one end, such as at an end closer to the funnel 18 of the catheter 10. A weakened portion 28 of the heat seal 26 may be provided with a tear-initiating notch 30, the weakened portion 28 helping to ensure a tear initiated at the notch 30 propagates predominantly linearly along the heat seal 26, substantially perpendicularly to the orientation of the catheter 10 as illustrated in the drawing (although it is recognized that the sleeved catheter 10 may be provided in any desired orientation within the foil wrapper 24, such as in a coiled arrangement (not shown) to reduce the overall footprint of the package). The foil wrapper 24 encloses liquid water exteriorly of the sleeve 12. The liquid water may be isolated from the sleeve 12 in any of various different ways, such as being provided in a saturated wick or length of fabric (not shown) that is either loose within the foil wrapper 24, secured to an inside wall of the foil wrapper 24, and/or provided in a separate cavity from the catheter 10 within the foil wrapper 24 formed by a liquid impermeable, vapor permeable barrier.

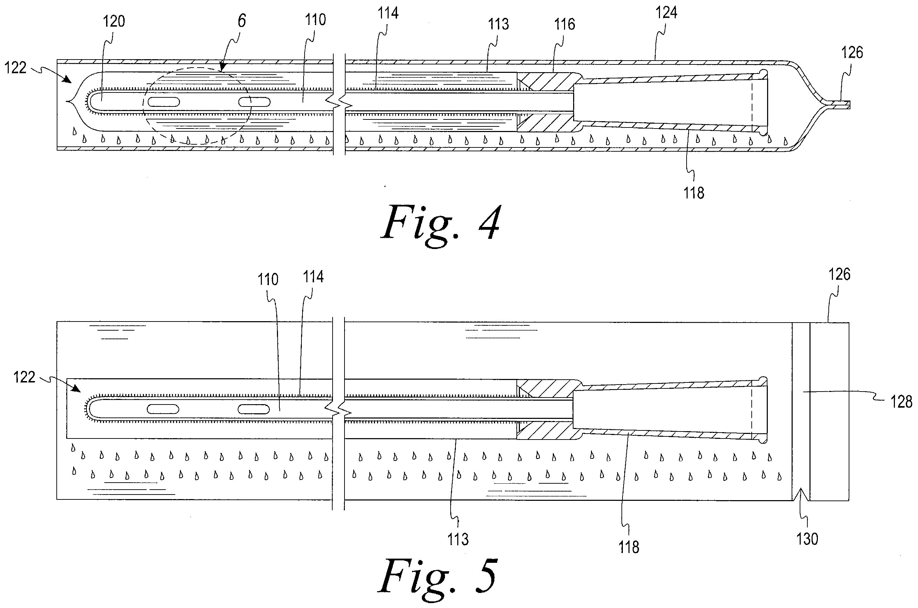

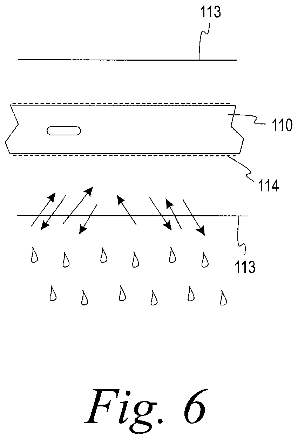

By way of comparison, figures 4-6 illustrate a sleeved catheter assembly 122, wherein a hydrophilic-coated catheter 110 is provided in a sleeve 113 that is liquid impermeable and vapor permeable. Elements in figures 4-6 correspond to like-numbered elements in the embodiment depicted in figures 1-3, increased by 100. Unlike sleeve 12, the sleeve 113 of the catheter assembly 122 is, as indicated by the arrows in figure 6, vapor permeable, in that water molecules travel not only from the exterior of the sleeve 113 to the interior of the sleeve 113, but can also travel from the interior of the sleeve 113 to the exterior of the sleeve 113. The sleeve 113 is made of soft polyurethane having a high surface energy, in that it does not mitigate the accumulation of water molecules on the sleeve's exterior.

As demonstrated by the following examples, it is found that providing a sleeve 12 of one or more membranes selected from the low surface energy family of polymers, such as PTFE and other fluoropolymers and/or polyolefins, it is possible to achieve acceptable hydration of the interior of the sleeve to activate the hydrophilic-coating 14 of the catheter 10, while providing a sleeve 12 that, upon withdrawal from the foil wrapper 24 for use, has an exterior that is drier to the touch, which is not the case for other high surface energy sleeves such as sleeve 113 made of, for example, soft polyurethane (PU) soft films.

### Example 1

A test was devised and performed to determine the extent to which wetness of a sleeve is reduced by the structural arrangement of the present disclosure. The test involved six initially dry hydrophilic-coated intermittent catheters, each placed in a respective initially dry sleeve constructed of a liquid impermeable GORETEX^{®} Medical Membrane, which is a material that is said to have preferential vapor permeability. The sleeve was constructed with the GORETEX^{®} Medical Membrane material oriented such that its water vapor permeability preferably permits the flow of water vapor in a direction from an exterior of the sleeve to an interior of the sleeve to a much greater extent than the extent to which water vapor can flow in a direction from the interior of the sleeve to the exterior of the sleeve. Low surface energy material is used in the formation of the sleeve in an effort, notwithstanding the permeability of the sleeve to water vapor, to avoid significant accumulation of water vapor on the exposed surface of the sleeve. Additionally, and as the control, two initially dry hydrophilic-coated intermittent catheters were placed in respective initially dry sleeves constructed of a liquid impermeable, vapor permeable membrane such as Mylan Medifilm^{®} 437 polyurethane membrane that is high in surface energy and generally bi-directionally vapor permeable. The samples were opened after 6 weeks conditioning in an oven kept at 40°C (104°F) and 75% relative humidity, and were tested for their sleeve wetness and catheter coating hydration, by measuring their coefficient of friction (CoF). After opening the packages, tiny droplets of water on sleeve of the first six samples were observed but they felt quite dry to the touch, unlike the other two samples, where the sleeve was felt to be wet and soaked in water. The coefficients of friction of these samples were measured using a Harland Friction tester model FTS5500 tester. The test included applying a 200g load to a 127mm section of a fully hydrated catheter. A mandrel is inserted into the catheter and it is then pulled through two pieces of silicon rubber with 60A Shore hardness at 10mm/s speed. The force required for pulling 80mm, out of a total length of 127mm, is then recorded using a universal tensile tester equipped with a 200N load cell. The CoF value is calculated from the ratio of applied to recorded loads when a steady state is reached. The CoF values obtained are tabulated below:

**Table 1**

| Sample No. | Initial CoF | Sleeve Dryness |
|---|---|---|
| 1 (in Gore-Tex^{®} sleeve) | 0.022 | Felt dry |
| 2 (in Gore-Tex^{®} sleeve) | 0.017 | Felt dry |
| 3 (in Gore-Tex^{®} sleeve) | 0.019 | Felt dry |
| 4 (in Gore-Tex^{®} sleeve) | 0.047 | Felt dry |
| 5 (in Gore-Tex^{®} sleeve) | 0.029 | Felt dry |
| 6 (in Gore-Tex^{®} sleeve) | 0.025 | Felt dry |
| Control (in PU sleeve) | 0.027 | Felt wet |
| Control (in PU sleeve) | 0.014 | Felt wet |

### Example 2

A second test was devised to verify and quantify the extent to which wetness of a sleeve is reduced by the structural arrangement of the present disclosure when the samples were conditioned for 6 weeks under a laboratory environment. According to this test, a first, initially dry, hydrophilic-coated intermittent catheter was placed in an initially dry sleeve constructed of a liquid impermeable, low surface energy GORETEX^{®} Medical Membrane material. A second initially dry, hydrophilic-coated intermittent catheter was placed in an initially dry liquid impermeable Mylan Medifilm 437^{®} polyurethane sleeve. Each of the dry sleeved catheters was then sealed in a separate liquid and vapor impermeable foil package just after a water-soaked fabric was provided in the foil package, such that the package had not begun producing a vapor atmosphere prior to introduction of the dry sleeved catheter. The two foil packages were placed in a store room and left for six weeks at a temperature of around 21°C (70°F), to cause them to produce a vapor atmosphere to which the sleeved catheters were exposed.

Immediately upon opening each of the foil packages, each of the sleeves was wiped with a dry, pre-weighed ply tissue to remove any excess moisture on the exterior of the sleeve, immediately after which the tissue was re-weighed to calculate the weight of moisture removed from the sleeve. Their CoF were also measured according to the above-described test method. These tests yielded the following results:

**Table 2**

| Sample No. | Initial CoF | Water on sleeve (g) |
|---|---|---|
| 1 (in Gore-Tex^{®} sleeve) | 0.024 | Not measured |
| 2 (in Gore-Tex^{®} sleeve) | 0.08 | Not measured |
| 3 (in Gore-Tex^{®} sleeve) | 0.03 | 0.045 |
| 4 (in Gore-Tex^{®} sleeve) | 0.034 | 0.061 |
| Control (in PU sleeve) | Not measured | 0.540 |

### Example 3

A third test was devised to quantify the extent to which wetness of a sleeve is reduced by the structural arrangement of the present disclosure when the samples are stored under harsher conditions in an oven at 40°C (104°F) and 75% relative humidity (RH) for four weeks. According to this test ten initially dry, hydrophilic-coated intermittent catheters were placed in an initially dry sleeve constructed from GORE-TEX^{®} Medical Membrane PTFE material. Similarly, ten initially dry, hydrophilic-coated intermittent catheters were placed in an initially dry liquid impermeable Mylan Medifilm 437 ^{®} polyurethane sleeve. Each of the dry sleeved catheters was then sealed in a separate liquid and vapor impermeable foil package just after a water-soaked fabric was provided in the foil package, such that the package had not begun producing a vapor atmosphere prior to introduction of the dry sleeved catheter. The two foil packages were placed in an oven at 40°C (104°F) and 75% RH, to cause them to produce a vapor atmosphere at a higher rate to which the sleeved catheters were exposed.

Immediately upon opening each of the foil packages, each of the sleeves was wiped with a dry, pre-weighed ply tissue to remove any excess moisture on the exterior of the sleeve, immediately after which the tissue was re-weighed to calculate the weight of moisture removed from the sleeve. This test yielded the following tabulated results:

**Table 3**

| | Sleeve wetness (g) | Sleeve wetness (g) |
|---|---|---|
| | Gore-Tex^{®} PTFE | Mylan^{®} PU |
| | Sleeve | |
| Average often samples | 0.08 | 0.45 |
| Standard Deviation | 0.05 | 0.21 |

Variations may be made to the specific embodiments described above that are still considered within the scope of the appended claims.

## Claims

1. A catheter assembly (22, 122) in a liquid impermeable, vapor impermeable package containing a liquid, the catheter assembly comprising:
a catheter (10, 110) having an insertable portion with a hydrophilic coating;
a liquid impermeable, vapor permeable sleeve (12, 113) having an exterior and an interior and surrounding at least the insertable portion of the catheter (10, 110), the sleeve (12) having a water vapor permeability of greater than about 300 g/m²/day and an exterior surface wherein a contact angle with water on the exterior surface of the sleeve is at or above about 100°; and
whereby a vapor produced from the liquid within the package can pass from the exterior to the interior of the sleeve (12, 113) to hydrate the hydrophilic coating, and wherein the liquid in the package is isolated from the sleeve (12, 113) in a manner permitting the liquid to at least partially change phase to produce the vapor within the package.

2. The catheter assembly of claim 1, wherein the sleeve (12, 113) comprises one or more membranes selected from a low surface energy polymer.

3. The catheter assembly of claim 2, wherein the one or more membranes are laminated to a non-woven fabric.

4. The catheter assembly of claim 3, wherein the non-woven fabric includes one or more of pressed polymeric fibers.

5. The catheter assembly of any one of the preceding claims, wherein the sleeve (12) comprises one or more of a fluoropolymer and a polyolefin.

6. The catheter assembly of any one of the preceding claims, wherein the sleeve (12) comprises PTFE.

7. The catheter assembly of any one of the preceding claims, wherein the sleeve (12) comprises a polyethylene.

8. The catheter assembly of any one of the preceding claims, wherein the liquid is water and the vapor permeability of the sleeve (12, 113) includes migration of water vapor molecules from the exterior of the sleeve (12, 113) to the interior of the sleeve (12, 113) to a greater extent than the extent to which the sleeve (12, 113) permits migration of water vapor molecules from the interior of the sleeve (12, 113) to the exterior of the sleeve (12, 113).

9. The catheter assembly of any one of the preceding claims, wherein the catheter includes a funnel (18, 118) having a collar (16, 116), the funnel (18, 118) disposed at an end of the catheter (10, 110) opposite a tip (20, 120) of the catheter (10, 110), and wherein the sleeve (12, 113) is sealed to the collar (16, 116) of the funnel (18, 118) of the catheter (10, 110).

10. The catheter assembly of any one of the preceding claims, wherein the water contact angle is between about 100° and about 120°.

11. The catheter assembly of any one of the preceding claims, wherein the sleeve (12, 113) has a water vapor permeability of greater than about 500 g/m²/day.

12. The catheter assembly of any one of the preceding claims, wherein the sleeve (12, 113) has a surface tension that is below about 40 mN/m or below about 36 mN/m.

13. The catheter assembly of any one of the preceding claims, wherein the sleeve (12, 113) has a surface tension that between about 20 to 30 mN/m or between about 15 to 36 mN/m.

## Patentansprüche

1. Katheterbaugruppe (22, 122) in einer flüssigkeitsundurchlässigen, dampfundurchlässigen Verpackung, die eine Flüssigkeit enthält, wobei die Katheterbaugruppe umfasst:
einen Katheter (10, 110), der einen einführbaren Abschnitt mit einer hydrophilen Beschichtung aufweist;
eine flüssigkeitsundurchlässige, dampfdurchlässige Hülse (12, 113), die eine Außenseite und eine Innenseite aufweist und mindestens den einführbaren Abschnitt des Katheters (10, 110) umgibt, wobei die Hülse (12) eine Wasserdampfdurchlässigkeit von mehr als etwa 300 g/m²/Tag und eine Außenfläche aufweist, bei der ein Kontaktwinkel mit Wasser auf der Außenfläche der Hülse bei oder über etwa 100° liegt; und
wobei ein Dampf, der von der Flüssigkeit in der Verpackung erzeugt wird, von der Außenseite in das Innere der Hülse (12, 113) gelangen kann, um die hydrophile Beschichtung zu hydratisieren, und wobei die Flüssigkeit in der Verpackung von der Hülse (12, 113) auf eine Weise isoliert ist, die es der Flüssigkeit erlaubt, zumindest teilweise die Phase zu wechseln, um den Dampf in der Verpackung zu erzeugen.

2. Katheterbaugruppe nach Anspruch 1, wobei die Hülse (12, 113) eine oder mehrere Membranen umfasst, die aus einem Polymer mit niedriger Oberflächenenergie ausgewählt sind.

3. Katheterbaugruppe nach Anspruch 2, wobei die eine oder mehrere Membranen auf einen Vliesstoff laminiert sind.

4. Katheterbaugruppe nach Anspruch 3, wobei der Vliesstoff eine oder mehrere gepresste Polymerfasern beinhaltet.

5. Katheterbaugruppe nach einem der vorhergehenden Ansprüche, wobei die Hülse (12) ein oder mehrere Fluorpolymere und Polyolefine umfasst.

6. Katheterbaugruppe nach einem der vorhergehenden Ansprüche, wobei die Hülse (12) PTFE umfasst.

7. Katheterbaugruppe nach einem der vorhergehenden Ansprüche, wobei die Hülse (12) ein Polyethylen umfasst.

8. Katheterbaugruppe nach einem der vorhergehenden Ansprüche, wobei die Flüssigkeit Wasser ist und die Dampfdurchlässigkeit der Hülse (12, 113) die Migration von Wasserdampfmolekülen von der Außenseite der Hülse (12, 113) in das Innere der Hülse (12, 113) in einem größeren Ausmaß beinhaltet als das Ausmaß, in dem die Hülse (12, 113) die Migration von Wasserdampfmolekülen aus dem Inneren der Hülse (12, 113) an die Außenseite der Hülse (12, 113) zulässt.

9. Katheterbaugruppe nach einem der vorhergehenden Ansprüche, wobei der Katheter einen Trichter (18, 118), der einen Kragen (16, 116) aufweist, beinhaltet, wobei der Trichter (18, 118) an einem Ende des Katheters (10, 110) gegenüber einer Spitze (20, 120) des Katheters (10, 110) angeordnet ist, und wobei die Hülse (12, 113) mit dem Kragen (16, 116) des Trichters (18, 118) des Katheters (10, 110) abgedichtet ist.

10. Katheterbaugruppe nach einem der vorhergehenden Ansprüche, wobei der Wasserkontaktwinkel zwischen etwa 100° und etwa 120° liegt.

11. Katheterbaugruppe nach einem der vorhergehenden Ansprüche, wobei die Hülse (12, 113) eine Wasserdampfdurchlässigkeit von mehr als etwa 500 g/m²/Tag aufweist.

12. Katheterbaugruppe nach einem der vorhergehenden Ansprüche, wobei die Hülse (12, 113) eine Oberflächenspannung aufweist, die unter etwa 40 mN/m oder unter etwa 36 mN/m liegt.

13. Katheterbaugruppe nach einem der vorhergehenden Ansprüche, wobei die Hülse (12, 113) eine Oberflächenspannung aufweist, die zwischen etwa 20 und 30 mN/m oder zwischen etwa 15 und 36 mN/m liegt.

## Revendications

1. Ensemble de cathéter (22, 122) dans un emballage imperméable aux liquides et imperméable à la vapeur contenant un liquide, l'ensemble cathéter comprenant :
un cathéter (10, 110) ayant une partie insérable avec un revêtement hydrophile ;
une manche (12, 113) imperméable aux liquides et perméable à la vapeur ayant un extérieur et un intérieur et entourant au moins la partie insérable du cathéter (10, 110), la manche (12) ayant une perméabilité à la vapeur d'eau supérieure à environ 300 g/m²/jour et une surface extérieure dans lequel un angle de contact avec l'eau sur la surface extérieure de la manche est supérieur ou égal à environ 100° ; et
moyennant quoi une vapeur produite à partir du liquide à l'intérieur de l'emballage peut passer de l'extérieur vers l'intérieur de la manche (12, 113) pour hydrater le revêtement hydrophile, et dans lequel le liquide dans l'emballage est isolé de la manche (12, 113) d'une manière permettant au liquide de changer au moins partiellement de phase pour produire la vapeur à l'intérieur de l'emballage.

2. Ensemble de cathéter selon la revendication 1, dans lequel la manche (12, 113) comprend une ou plusieurs membranes choisies parmi un polymère à tension de surface réduite.

3. Ensemble de cathéter selon la revendication 2, dans lequel les une ou plusieurs membranes sont laminées sur un tissu non tissé.

4. Ensemble de cathéter selon la revendication 3, dans lequel le tissu non tissé comporte une ou plusieurs fibres polymères pressées.

5. Ensemble de cathéter selon l'une quelconque des revendications précédentes, dans lequel la manche (12) comprend un ou plusieurs parmi un fluoropolymère et une polyoléfine.

6. Ensemble de cathéter selon l'une quelconque des revendications précédentes, dans lequel la manche (12) comprend du PTFE.

7. Ensemble de cathéter selon l'une quelconque des revendications précédentes, dans lequel la manche (12) comprend un polyéthylène.

8. Ensemble de cathéter selon l'une quelconque des revendications précédentes, dans lequel le liquide est de l'eau et la perméabilité à la vapeur de la manche (12, 113) comporte la migration des molécules de vapeur d'eau de l'extérieur de la manche (12, 113) vers l'intérieur de la manche (12, 113) dans une mesure supérieure à la mesure dans laquelle la manche (12, 113) permet la migration des molécules de vapeur d'eau de l'intérieur de la manche (12, 113) vers l'extérieur de la manche (12, 113).

9. Ensemble de cathéter selon l'une quelconque des revendications précédentes, dans lequel le cathéter comporte un entonnoir (18, 118) ayant une collerette (16, 116), l'entonnoir (18, 118) étant disposé à une extrémité du cathéter (10, 110) à l'opposé d'un embout (20, 120) du cathéter (10, 110), et dans lequel la manche (12, 113) est scellée à la collerette (16, 116) de l'entonnoir (18, 118) du cathéter (10, 110).

10. Ensemble de cathéter selon l'une quelconque des revendications précédentes, dans lequel l'angle de contact avec l'eau est compris entre environ 100° et environ 120°.

11. Ensemble de cathéter selon l'une quelconque des revendications précédentes, dans lequel la manche (12, 113) a une perméabilité à la vapeur d'eau supérieure à environ 500 g/m²/jour.

12. Ensemble de cathéter selon l'une quelconque des revendications précédentes, dans lequel la manche (12, 113) a une tension de surface qui est inférieure à environ 40 mN/m ou inférieure à environ 36 mN/m.

13. Ensemble de cathéter selon l'une quelconque des revendications précédentes, dans lequel la manche (12, 113) a une tension de surface comprise entre environ 20 et 30 mN/m ou comprise entre environ 15 et 36 mN/m.
